# EUROPEAN PATENT SPECIFICATION

(11) **EP 3 460 474 B1**
(45) Date of publication and mention of the grant of the patent: **06.04.2022**
(21) Application number: 17799494.4
(22) Date of filing: 19.05.2017
(51) Int. Cl.: G01N 33/543, C12Q 1/68, G01N 33/58

(54) **METHOD AND KIT FOR TARGET MOLECULE DETECTION**
VERFAHREN UND KIT ZUR ZIELMOLEKÜLDETEKTION
PROCÉDÉ ET KIT DE DÉTECTION DE MOLÉCULE CIBLE

(30) Priority: 19.05.2016 JP 2016100231; 13.12.2016 JP 2016241023
(43) Date of publication of application: 27.03.2019
(73) Proprietor: Toppan Printing Co., Ltd., Tokyo 110-0016 (JP)
(72) Inventor: MAKINO, Yoichi, Tokyo 110-0016 (JP); HIRASE, Takumi, Tokyo 110-0016 (JP); ARAI, Noriaki, Tokyo 110-0016 (JP); NOJI, Hiroyuki, Saitama-shi Saitama 336-0963 (JP)
(74) Representative: Plasseraud IP
(86) International application number: PCT/JP2017/018782
(87) International publication number: WO 2017/200070

(56) References cited:
- WO-A1-2014/124046
- WO-A1-2015/175856
- WO-A1-2015/200139
- WO-A1-2016/047068
- WO-A2-2012/071428
- JP-A- 2005 528 103
- JP-A- 2010 525 792
- JP-A- 2015 230 280
- JP-B2- 4 111 984
- JP-B2- 5 551 798
- US-A1- 2013 323 729
- US-A1- 2014 248 710
- HANAMI, TAKESHI et al.: "Eprobe mediated real- time PCR monitoring and melting curve analysis", PLOS ONE, vol. 8, no. 8, 7 August 2013 (2013-08-07), XP002755207,

## Description

### [Technical Field]

The present invention relates to a method and kit for target molecule detection.

This application is based on and claims the benefit of priority from Japanese Patent Application No. 2016-100231 filed on May 19, 2016, and Japanese Patent Application No. 2016-241023 filed on December 13, 2016.

### [Background Art]

Early detection of disease and prediction of the effects of medication are performed by quantitatively detecting a target molecule in a biological sample. Conventionally, protein quantification has been performed by enzyme-linked immunosorbent assay (ELISA) or the like, and nucleic acid quantification has been performed by the real-time PCR method or the like.

In recent years, there is an increasing need for detecting target molecules more accurately for the purpose of, for example, finding disease earlier. As a method for accurately detecting a target molecule, for example, a technique for performing an enzyme reaction in a large number of minute compartments is described in PTL 1, PTL 2, NPL 1 and the like.

These methods are called digital measurement.

In digital measurement, a sample solution is divided into an extremely large number of minute solutions. Then, a signal from each minute solution is binarized, only whether target molecules are present is determined, and the number of target molecules is measured. With digital measurement, the detection sensitivity and the quantitativeness can be markedly improved compared with conventional methods such as ELISA, the real-time PCR method and the like.

In digital measurement, the presence of target molecules may be detected using a specific binding substance such as a monoclonal antibody that binds to the target molecules. However, in the detection of target molecules using a specific binding substance, the specific binding substance may cause a misrecognition of a homologous epitope on a protein having a structure similar to the target molecules, or a misrecognition such as nonspecific recognition, and the like, resulting in a decrease in the detection accuracy of the substance.

In order to solve this problem, PTL 3, for example, describes a method for detecting target molecules. This method includes a complex formation procedure, a detection procedure, and an analysis procedure. In the complex formation procedure, target molecules, a carrier modified by a first antibody that specifically binds to the target molecule, and two or more second antibodies that are labeled with enzymes having substrate cleavage activity and different substrate specificities, and specifically bind to the target molecules, are made to react to form a complex that includes the first antibody, the target molecules and the second antibody on the carrier; in the detection procedure, two or more substrates each having a cleavage site to be cleaved by the enzymes, with a fluorescent substance being bonded to one end side of the cleavage site and a quenching substance being bonded to the other end side, the fluorescent wavelengths of the fluorescent substances being different from each other, are made to react with the complex, and the fluorescent light emitted from the fluorescent substances is detected; in the analysis procedure, detection signals of two or more fluorescent lights having different fluorescent wavelengths are processed as detection signals of the target molecules.

### [Citation List]

### [Patent Literature]

PTL 1: JP 5551798 B
PTL 2: JP2014-503831 A
PTL 3: WO2016/047068

### [Non-Patent Literature]

NPL 1: Kim S. H., et al., Large-scale femtoliter droplet array for digital counting of single biomolecules., Lab on a Chip, 12 (23), 4986-4991, 2012.

WO 2014/124046, US 2013/323729, WO 2012/071428, US 2014/248710 disclose methods for detecting target molecules using nucleic acid-labeled binding substances.

### [Summary of the Invention]

### [Technical Problem]

However, the method described in PTL 3 has room for improvement. For example, in the method described in PTL 3, a signal amplification reaction is not included in the process in which an enzyme cleaves a substrate and fluorescence is detected. In addition, the number of enzyme molecules that can be labeled to an antibody molecule depends on the size of the molecule, but is often several per antibody molecule. Consequently, in order to detect fluorescence by the method described in PTL 3, a reaction time of at least several tens of minutes is necessary.

Therefore, an object of the present invention is to provide a technique capable of quickly and accurately detecting a target molecule.

Moreover, another object of the present invention is to provide a technique capable of accurately detecting a target molecule even in the case where a non-specific binding substance causes misrecognition of a target molecule.

### [Solution to Problem]

The method for detecting a target molecule according to a first aspect of the present invention is provided in the independent claim 1. Preferred embodiments are defined in the dependent claims.

### [Advantageous Effects of Invention]

According to the above-described aspects of the present invention, it is possible to provide a technique capable of rapidly and accurately detecting a target molecule.

In addition, according to the above-described aspects of the present invention, it is possible to provide a technique capable of accurately detecting a target molecule even in the case where a non-specific binding substance causes misrecognition of a target molecule.

### [Brief Description of Drawings]

FIG. 1A is a schematic cross-sectional view illustrating a conventional method of detecting a target molecule in a microscopic compartment.
FIG. 1B is a schematic cross-sectional view illustrating a conventional method of detecting a target molecule in a microscopic compartment.
FIG. 2 is a schematic cross-sectional view for illustrating detection of a target molecule by digital measurement.
FIG. 3A is a schematic cross-sectional view illustrating a first embodiment of a method of detecting a target molecule in a microscopic compartment.
FIG. 3B is a schematic cross-sectional view illustrating the first embodiment of a method of detecting a target molecule in a microscopic compartment.
FIG. 4A is a schematic cross-sectional view illustrating a second embodiment of a method of detecting a target molecule in a microscopic compartment.
FIG. 4B is a schematic cross-sectional view illustrating the first embodiment of a method of detecting a target molecule in a microscopic compartment.
FIG. 5A is a diagram for illustrating an example of a combination of a specific binding substance and a label capable of simultaneously detecting a plurality of types of target molecules.
FIG. 5B is a diagram for illustrating an example of a combination of a specific binding substance and a label capable of simultaneously detecting a plurality of types of target molecules.
FIG. 5C is a diagram for illustrating an example of a combination of a specific binding substance and a label capable of simultaneously detecting a plurality of types of target molecules.
FIG. 5D is a diagram for illustrating an example of a combination of a specific binding substance and a label capable of simultaneously detecting a plurality of types of target molecules.
FIG. 5E is a diagram for illustrating an example of a combination of a specific binding substance and a label capable of simultaneously detecting a plurality of types of target molecules.
FIG. 5F is a diagram for illustrating an example of a combination of a specific binding substance and a label capable of simultaneously detecting a plurality of types of target molecules.
FIG. 5G is a diagram for illustrating an example of a combination of a specific binding substance and a label capable of simultaneously detecting a plurality of types of target molecules.
FIG. 5H is a diagram for illustrating an example of a combination of a specific binding substance and a label capable of simultaneously detecting a plurality of types of target molecules.
FIG. 6 is a graph illustrating the results of Example 2.
FIG. 7 is a graph illustrating the results of a Comparative Example 2.
FIG. 8 illustrates the results of exosome detection in a culture supernatant by a digital Invader method according to Example 4.
FIG. 9 illustrates the results of exosome detection in serum by a digital Invader method according to Example 4.

### [Description of Embodiments]

Hereinafter, embodiments of the present invention will be described in detail while in some cases referring to the accompanying drawings. Note that in the drawings, the same or corresponding parts are denoted by the same or corresponding reference numerals, and redundant description is omitted. Incidentally, there are portions where the dimensional ratios in the respective figures are exaggerated for the sake of explanation, and do not necessarily coincide with the actual size ratios.

### [Conventional Detection Method]

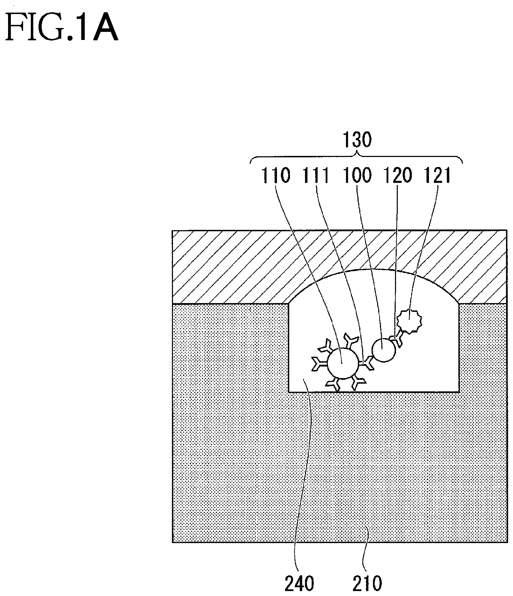

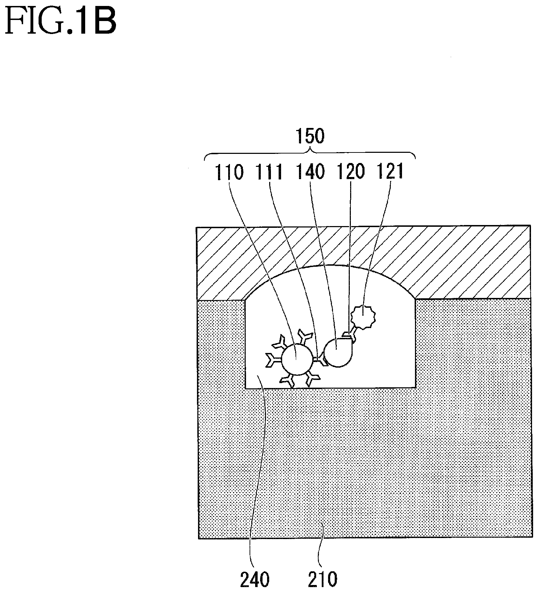

First, a typical example of a conventional method for detecting a target molecule will be described. FIGs. 1A and 1B are schematic cross-sectional views illustrating a conventional method of detecting a target molecule in a minute compartment. In FIG. 1A, a complex 130 of a target molecule 100, a target molecule capturing particle 110, and a specific binding substance 120 for binding to a target molecule 100 is accommodated in a well 240 formed on the surface of a substrate 210. A specific binding substance 111 is immobilized on the target molecule capturing particle 110. In addition, the specific binding substance 120 has a label (labeling molecule) 121. In FIG. 1A, the specific binding substance 111 and the specific binding substance 120 that are immobilized on the target molecule capturing particle 110 correctly recognize an epitope of the target molecule 100.

However, in FIG. 1B, at least one of the specific binding substance 111 or the specific binding substance 120 erroneously recognizes a molecule 140 that is not the target molecule, and forms the complex 150.

Here, in the case where it is determined that the target molecule 100 is present with the presence of the label 121 as an indicator, the specific binding substance 120 has the label 121, so it is determined that the target molecule 100 is present.

In this way, according to the conventional detection method, when a non-specific binding substance causes misrecognition of a target molecule, the target molecule may not be accurately detected.

Causes of misrecognition of a target molecule by a specific binding substance include the presence of a homologous epitope on a protein or peptide having a structure similar to the target molecule, with such a misrecognition occurring at a constant frequency. In ELISA, which is a conventional detection method, there is a small measurement error even when only a few specific binding substances cause misrecognition; however, in digital measurement it is necessary to accurately measure a low concentration region of the target molecules, so misrecognition by antibodies affects measurement errors.

### [Target Molecule Detection Method]

One embodiment of the present disclosure (not claimed) provides a method for detecting a target molecule, the method including a step of binding a plurality of types of specific binding substances, which each recognize a different epitope, to a target molecule, a step of detecting each of the plurality of types of specific binding substances, and a step of determining that the target molecule is present when two or more types of the plurality of types of specific binding substances are detected. Hereinafter, a detection method according to the present embodiment will be described.

### (Step of Binding a Plurality of Types of Specific Binding Substances to a Target Molecule)

First, a plurality of types of specific binding substances, which each recognize a different epitope, are bound to a target molecule. This step can be performed, for example, by bringing the target molecule and the specific binding substance into contact with each other in a sample tube. Here, the number of types of specific binding substances is two or more, but may be three or more, four or more, or five or more.

### <Target Molecule>

The target molecule is not limited, and any molecule can be detected. More specifically, examples may include a disease marker protein in a biological sample, a specific compound in an environmental sample, and the like. The biological sample is not limited, and examples thereof may include serum, plasma, urine, and the like. In addition, examples of environmental samples may include river water, factory wastewater, and the like.

### <Specific Binding Substance>

Examples of specific binding substances include antibodies, antibody fragments, aptamers, and the like. Antibodies can be prepared, for example, by immunizing an animal such as a mouse or the like with a target molecule or a fragment of a target molecule as an antigen. Alternatively, for example, antibodies can be prepared by screening a phage library. Examples of antibody fragments include Fab, F (ab') 2, Fab', single chain antibody (scFv), disulfide stabilized antibody (dsFv), dimeric V region fragment (Diabody), peptide including CDR, and the like. The antibody may be a monoclonal antibody or a polyclonal antibody. Alternatively, the antibody may be a commercially available antibody.

An aptamer is a substance having a specific binding ability for binding to a target molecule. Examples of aptamers include nucleic acid aptamers, peptide aptamers, and the like. A nucleic acid aptamer having a capability of specifically binding to a target molecule can be selected, for example, by the systematic evolution of ligands by the exponential enrichment (SELEX) method, or the like. Peptide aptamers having a capability of specifically binding to a target molecule can be selected, for example, by a two-hybrid method using yeast, or the like.

### (Step of Detecting Each of the Plurality of Types of Specific Binding Substances)

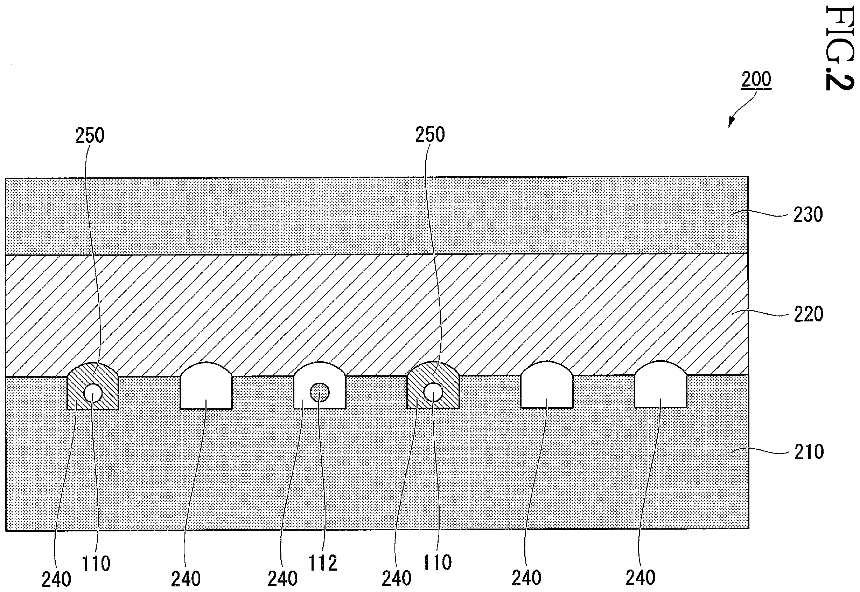

Then, a plurality of types of the specific binding substances are respectively detected. FIG. 2 is a schematic cross-sectional view for illustrating detection of a target molecule by digital measurement using a detection device 200 as an example.

As illustrated in FIG. 2, the detection device 200 includes a substrate 210, a flow channel 220, and a lid 230; and a plurality of wells 240 are formed on the surface of the substrate 210.

First, target molecules are accommodated in the wells 240. The method of accommodating the target molecules in the wells 240 is not limited; for example, by bringing a target molecule capturing particle on which a specific binding substance with respect to a target molecule is immobilized into contact with the above-described target molecule, the target molecule capturing particle captures the molecule, and by subsequently introducing the target molecule capturing particle into the flow channel 220 of the detection device 200, the target molecule capturing particle is accommodated in the well 240.

Here, the target molecule capturing particle, the target molecule, and the above-described plurality of types of specific binding substances may be brought into contact in any order. For example, the target molecule capturing particle, the target molecule, and plurality of types of specific binding substances may be brought into contact at the same time. Alternatively, the plurality of types of specific binding substances may be brought into contact after bringing the target molecule capturing particle into contact with the target molecule. Alternatively, the target molecule capturing particle may be further brought into contact after bringing the target molecule into contact with the plurality of types of specific binding substances.

Here, preferably the target molecule capturing particle and the target molecule are brought into contact with each other under a condition that 0 or 1 target molecule is captured by one target molecule capturing particle. Furthermore, preferably one well 240 is configured to accommodate 0 or 1 target molecule capturing particles. As a result digital measurement becomes possible. In other words, in the detection method according to the present embodiment, the detection of a target molecule may be performed in one molecule units.

### <Target Molecule Capturing Particle>

The target molecule capturing particle is a particle capable of capturing a target molecule. For example, there is the above-mentioned target molecule capturing particle on which a specific binding substance with respect to a target molecule is immobilized; however, the present invention is not limited to this.

The particle is not limited, and a polymer particle, magnetic particle, glass particle, and the like can be used. The particle is preferably a particle subjected to a surface treatment in order to avoid nonspecific adsorption.

The method of immobilizing the specific binding substance on the surface of the particle is not limited, and it is possible to use a method of physical adsorption, a method of chemical bonding, a method using avidin-biotin binding, a method of using a bond between protein G or protein A to an antibody, or the like. Of these methods, chemical bonding is preferred because of its high stability.

As the method by physical adsorption, there is a method of immobilizing a specific binding substance on the particle surface by hydrophobic interaction or electrostatic interaction. As the method by chemical bonding, there is a method using a crosslinking agent. For example, in the case where the surface of the particle has a hydroxyl group, the carboxyl group of the specific binding substance is reacted with a crosslinking agent to obtain an active ester, and then the hydroxyl group and the ester group are reacted, whereby the specific binding substance can be immobilized on the particle surface. It is also preferable to provide a spacer between the specific binding substance and the particle surface so as not to inhibit the recognition ability of the specific binding substance to recognize the target molecule.

### (Example of the Step of Binding a Plurality of Types of Specific Binding Substances)

In FIG. 2, the wells 240 accommodate zero or one target molecule capturing particle 110 or 112. In addition, the flow channel 220 is filled with a sealing liquid, and the solution accommodated in each well 240 forms droplets (independent reaction chambers). In FIGs. 1A and 1B, the target molecule capturing particle 110 captures one target molecule, and the target molecule capturing particle 112 does not capture a target molecule. In addition, a plurality of types of specific binding substances, which each recognize a different epitope, are bound to the target molecule.

Then, the above-mentioned a plurality of types of specific binding substances are respectively detected. The method for detecting each of the plurality of types of specific binding substances is the Invader method; for example, the plurality of types of specific binding substances may be labeled so as to be distinguishable from each other, and by detecting each of the respective types of labels, it is possible to detect each of the plurality of types of specific binding substances bound to a target molecule.

A specific binding substance may be directly labeled or indirectly labeled. For example, particles on which a specific binding substance and a labeling molecule are immobilized may be used as a labeled specific binding substance. Here, particles similar to those described above can be used as the particles.

Examples of labeling molecules that can be distinguished from each other include, for example, mutually different nucleic acid fragments, mutually different enzymes, mutually different fluorescent dyes, and the like. These labels may be used in combination. Specific binding substances can be detected utilizing the difference in the base sequence of the nucleic acid fragments. Specific binding substances are detected by converting differences in the nucleotide sequence of nucleic acid fragments into signals using the Invader method. The method for labeling a specific binding substance with a nucleic acid fragment is not limited, and examples may include a method of utilizing avidin-biotin binding, a method of using a crosslinking agent, and the like.

The signal is not limited and, for example, fluorescence, luminescence, color development, pH change, potential change, and the like can be used.

In an example that is not claimed, in the case where the labeling molecules are different enzymes, a specific binding substance can be detected by reacting a fluorescent substrate or the like corresponding to respective enzymes. Moreover, in the case where the labeling molecules are different fluorescent dyes, specific binding substances can be directly detected by single molecule fluorescence detection or the like.

In the case where the step of detecting a specific binding substance includes a signal amplification reaction, the detection sensitivity can be improved, and the time required for detection can be shortened. As an example of a signal amplification reaction, there is, for example, an enzymatic reaction.

In the case where a plurality of types of specific binding substances are bound to one target molecule and a plurality of types of specific binding substances are respectively detected, by determining the target molecule is present when two or more types, preferably, all types of specific binding substances are detected, the target molecule can be detected accurately. This is because the probability of erroneous recognition by two or more types of the plurality of specific binding substances is low.

All of the plurality of types of specific binding substances may be labeled with nucleic acid fragments. Moreover, a plurality of types of specific binding substances are detected using the Invader method. In this case, it is possible to detect the difference in the nucleotide sequence of all the nucleic acid fragments by using one type of enzyme (Cleavase). According to such a method, since only one type of enzyme is used, it is possible to adjust the reaction system to an optimum condition for the enzyme to be used.

In addition, since the Invader method includes a signal amplification reaction, the time required to detect a plurality of types of specific binding substances can be shortened to be within several minutes.

In addition, for example, in the case of labeling a nucleic acid fragment to an antibody molecule, several to several tens of nucleic acid fragments can be labeled per antibody molecule, for example, since nucleic acid fragments are relatively small molecules. By increasing the number of nucleic acid fragments per antibody molecule, the time required to detect a plurality of types of specific binding substances can be further shortened. In the case where a nucleic acid fragment is labeled to an antibody molecule, several to several hundreds of nucleic acid fragments may be labeled per antibody molecule.

According to the present embodiment, for example, the time for a nucleic acid detection reaction (for example, Invader reaction) is about 1 minute to 30 minutes, and rapid nucleic acid detection is possible.

On the other hand, in the method described in PTL 3, for example, two types of enzymes having mutually different substrate specificities are used. Therefore, in the case where optimal reaction conditions of these enzymes are different, it is difficult to adjust the reaction system to optimum conditions for all of the enzymes to be used.

Moreover, in the method described in PTL 3, a signal amplification reaction is not included in a process in which an enzyme cleaves a substrate and fluorescence is detected. In addition, the number of enzyme molecules that can be labeled to an antibody molecule depends on the size of the molecule, but is often several per antibody molecule. Consequently, in order to detect fluorescence by the method described in PTL 3, a reaction time of at least several tens of minutes is necessary.

In FIG. 2, each well 240 accommodates zero or one target molecule capturing particle. Signals 250 derived from a plurality of types of specific binding substances are detected from the wells 240 accommodating the target molecule capturing particles 110 that capture the target molecules. The signals 250 indicate that all of the types of the plurality of types of specific binding substances are detected.

On the other hand, a signal 250 is not detected from a well 240 that does not accommodate a target molecule capturing particle or a well 240 that accommodates a target molecule capturing particle 112 that does not capture a target molecule. Therefore, by counting the number of wells 240 in which a signal 250 is detected, the concentration of target molecules can be accurately measured.

Here, the structure of the detection device 200 will be described in more detail. The wells 240 are aligned on the surface of the substrate 210 and may have, for example, a closed-bottom columnar shape having a diameter of 5 µm and a depth of 5 µm.

For example, the wells 240 may be arranged in a lattice shape along each side of a rectangular region having a 5 mm length and 5 mm width on the surface of the substrate 210. The size of the gap between the wells 240 may be set according to the resolution capable of independently detecting the signal of each well 240.

The volume of the wells 240 can be appropriately set; however, in the case where the step of detecting the specific binding substance accompanies a signal amplification reaction, the reaction time until signal detection becomes possible is shorter the smaller the volume of the wells 240. For example, the volume of wells 240 may be 100 picoliters or less.

For example, for the purpose of shortening the time required to generate a signal having sufficient intensity, the volume of the wells 240 may be set based on the amount of liquid in which the number of target molecules is one or fewer per well.

When doing this, the detection device 200 may be configured so that the size of one well 240 is three times or less the volume of a target molecule.

Moreover, besides producing the detection device 200 so that the one or fewer target molecule is introduced into one well, the detection device 200 may be produced so that one or fewer of another target molecule (second target molecule) having a target molecule (a first target molecule) (for example, exosome or the like as a second target molecule having a protein as a first target molecule to be described later) is introduced into one well. Furthermore, besides producing the detection device so that there is one or fewer target molecule in one well, it is also possible to introduce target molecules into the wells so that there is one or fewer target molecule in one well by diluting the concentration of target molecules.

After preparing a detection device 200 that includes a substrate 210 having a plurality of wells 240, and after the step of binding the plurality of types of specific binding substances to a plurality of first target molecules, the method may further include a step of introducing one second target molecule (a second target molecule having a first target molecule) into one of the plurality of wells.

In this case, the volume of the well 240 may be 100 picoliters or less.

In addition, the size of the well 240 may be configured so that one second target molecule is introduced depending on the second target molecule selected; for example, the detection device 200 may be configured so the size of one well 240 is three times or less the volume of the second target molecule.

For example, by implementing a configuration in which first target molecules or second target molecules are individually introduced into wells of one device (for example, a configuration in which one first target molecule is introduced into one well, or one second target molecule is introduced into one well), target molecules (first target molecule, second target molecule) can be accurately detected without the interference by fluorescence signals of labeled nucleic acids, even though a large number of fluorescently labeled nucleic acids are present.

The substrate 210 is formed using a resin, for example. The type of resin is not limited; however, it is preferable to use a resin that is resistant to a reagent and a sealing solution used in forming droplets. Also, in the case where fluorescent observation of a signal is performed, it is preferable to select a resin with less autofluorescence. Such a resin may be a cycloolefin polymer (COP), cycloolefin copolymer (COC), fluororesin or the like.

### (First Embodiment)

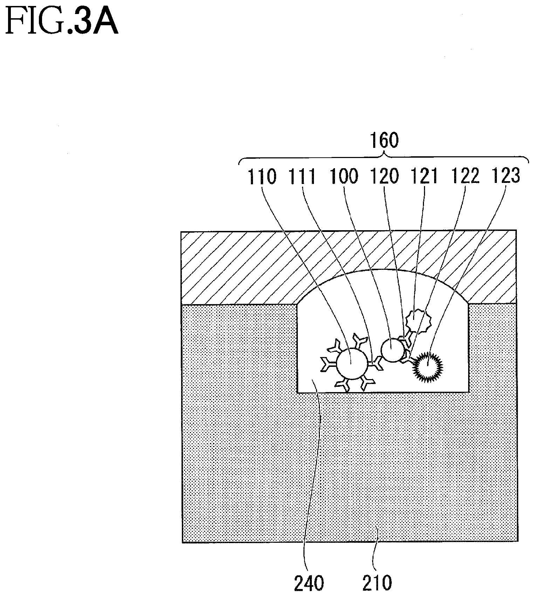

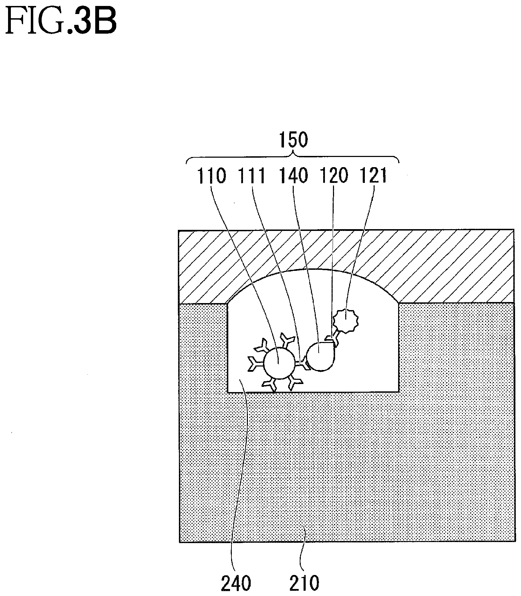

A method for detecting a target molecule according to a first embodiment will be described. FIGs. 3A and 3B are schematic cross-sectional views illustrating a method according to the present embodiment for detecting a target molecule inside a minute compartment. In FIG. 3A, a complex 160 of a target molecule 100, a target molecule capturing particle 110, a specific binding substance 120 for binding to the target molecule 100, and a specific binding substance 122 which recognizes an epitope different from that recognized by the specific binding substance 120, is accommodated in a well 240 formed on the surface of the substrate 210. The specific binding substance 120 has a label 121. The specific binding substance 122 has a label 123. In FIG. 3A, the specific binding substance 111 immobilized on the target molecule capturing particle 110, the specific binding substance 120, and the specific binding substance 122 correctly recognize an epitope of the target molecule 100. The marker 121 and the marker 123 are distinguishable from each other. For example, the label 121 and the label 123 can generate fluorescence signals of different fluorescence wavelengths.

On the other hand, in FIG. 3B, at least one of the specific binding substance 111 or the specific binding substance 120 erroneously recognizes the molecule 140 which is not the target molecule, and forms a complex 150.

Here, since the specific binding substance 122 is not bound to the molecule 140, there is no label 123 in the complex 150. Therefore, in the complex 150, although a signal derived from the label 121 is detected, a signal derived from the label 123 is not detected.

Therefore, the specific binding substance in the complex 150 can be determined as misrecognizing the target molecule. As a result, by measuring only the number of wells accommodating the complex 160 which correctly recognizes the target molecule, it is possible to accurately detect the target molecule even in the case where misrecognition of the target molecule by the non-specific binding substance may occur.

### (Second Embodiment)

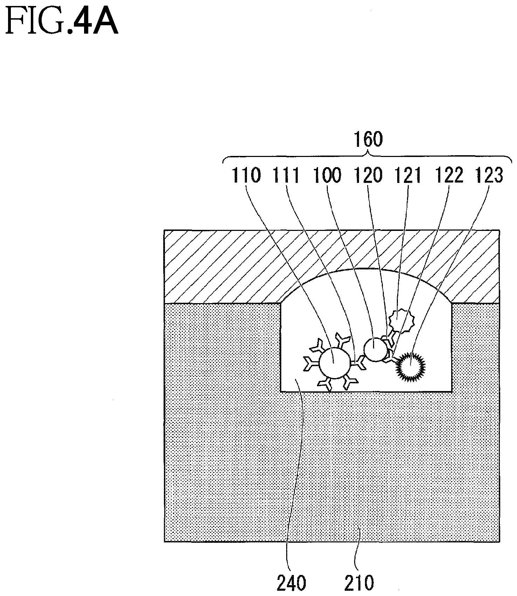

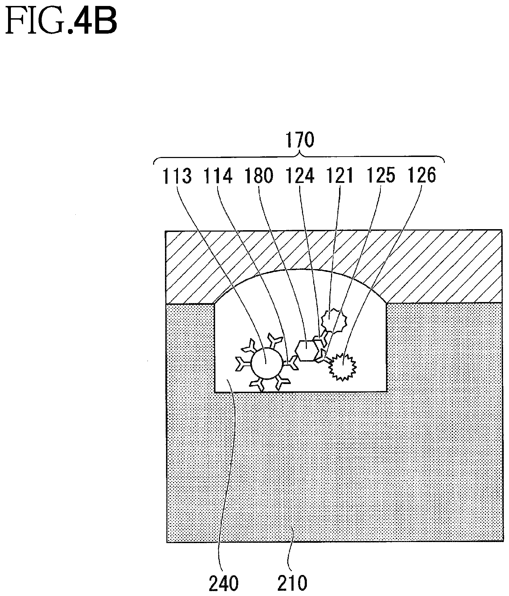

A method for detecting a target molecule according to a second embodiment will be described. The detection method according to this embodiment is a method for accurately detecting a plurality of types of target molecules (a plurality of types of first target molecules) at the same time. FIGs. 4A and 4B are schematic cross-sectional views illustrating a method according to this embodiment for detecting a target molecule in a microscopic compartment. In FIG. 4A, a complex 160 of a target molecule 100, a target molecule capturing particle 110, a specific binding substance 120 for binding to the target molecule 100, and a specific binding substance 122 which recognizes an epitope different from that recognized by the specific binding substance 120, is accommodated in a well 240 formed on the surface of the substrate 210. The specific binding substance 120 has a label 121. The specific binding substance 122 has a label 123. In FIG. 4A, the specific binding substance 111 immobilized on the target molecule capturing particle 110, the specific binding substance 120 and the specific binding substance 122 correctly recognize an epitope of the target molecule 100. The marker 121 and the marker 123 are distinguishable from each other. For example, the label 121 and the label 123 can generate fluorescence signals of different fluorescence wavelengths. In this embodiment, for example, when fluorescence signals respectively obtained from a plurality of labels are visible lights, it is possible to perform detection (multicolor detection) using various colors.

In addition, in FIG. 4B, a complex 170 of the target molecule 180, the target molecule capturing particle 113, the specific binding substance 124 for binding to the target molecule 180, and the specific binding substance 125 which recognizes an epitope different from that recognized by the specific binding substance 124, is accommodated in a well 240 formed on the surface of the substrate 210. The specific binding substance 124 has a label 121. The specific binding substance 125 has a label 126. In FIG. 4B, the specific binding substance 114 immobilized on the target molecule capturing particle 113, the specific binding substance 124 and the specific binding substance 125 correctly recognize an epitope of the target molecule 180. The label 121 and the label 126 are distinguishable from each other. For example, the label 121 and the label 126 can generate fluorescence signals of fluorescence wavelengths that differ from each other.

Here, in FIGs. 4A and 4B, the detected signal can be identified. In other words, from the complex 160 in FIG. 4A, a signal derived from the label 121 and a signal derived from the label 123 are detected. In addition, from the complex 170 in FIG. 4B, a signal derived from the label 121 and a signal derived from the label 126 are detected.

Therefore, by the detection method according to the present embodiment, it is possible to identify the target molecule 100 and the target molecule 180 mixed and present in the sample, and simultaneously detect the respective target molecules 100, 180 with high accuracy.

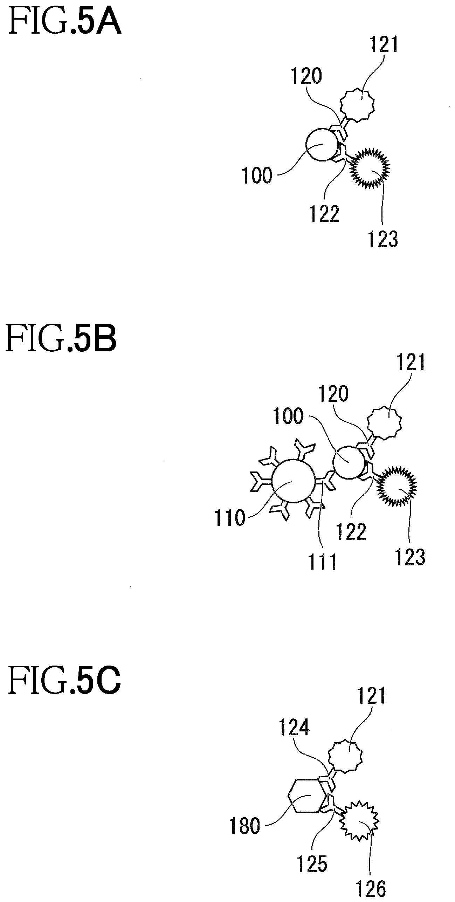

Examples of combinations of specific binding substances and labels in the case of simultaneously detecting a plurality of types of target molecules will be described. FIGs. 5A to 5H are diagrams for illustrating examples of combinations of specific binding substances and labels capable of simultaneously detecting a plurality of types of target molecules.

In FIG. 5A, a specific binding substance 120 and a specific binding substance 122 are bound to a target molecule 100 in order to detect the target molecule 100. The specific binding substance 120 has a label 121. In addition, the specific binding substance 122 has a label 123. The specific binding substance 120 and the specific binding substance 122 each recognize a different epitope. In addition, the label 121 and the label 123 can be distinguished from each other.

FIG. 5B is a diagram illustrating an example in which a target molecule capturing particle 110 is further bonded to the complex in FIG. 5A. A specific binding substance 111, which recognizes an epitope different from epitopes recognized by the specific binding substance 120 and the specific binding substance 122, is immobilized on the target molecule capturing particle 110.

As described above, by using a target molecule capturing particle, it becomes easy to accommodate, for example, 0 molecules or 1 molecule of the target molecule in each well forming a micro compartment, and to perform digital measurement.

In FIG. 5C, a specific binding substance 124 and a specific binding substance 125 are bound to a target molecule 180 in order to detect the target molecule 180. The specific binding substance 124 has a label 121. Moreover, the specific binding substance 125 has a label 126. The specific binding substance 124 and the specific binding substance 125 each recognize a different epitope. In addition, the label 121 and the label 126 can be distinguished from each other.

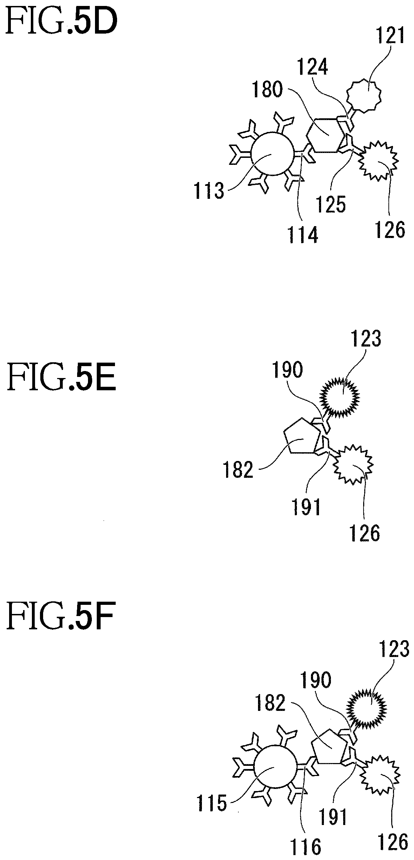

FIG. 5D is a diagram illustrating an example in which a target molecule capturing particle 113 is further bonded to the complex in FIG. 5C. A specific binding substance 114, which recognizes an epitope different from epitopes recognized by the specific binding substance 124 and the specific binding substance 125, is immobilized on a target molecule capturing particle 113.

As described above, by using a target molecule capturing particle, it becomes easy to accommodate, for example, 0 molecules or 1 molecule of the target molecule in each well forming a micro compartment, and to perform digital measurement.

In FIG. 5E, a specific binding substance 190 and a specific binding substance 191 are bound to a target molecule 182 in order to detect the target molecule 182. The specific binding substance 190 has a label 123. Moreover, the specific binding substance 191 has a label 126. The specific binding substance 190 and the specific binding substance 191 each recognize a different epitope. In addition, the label 123 and the label 126 can be distinguished from each other.

FIG. 5F is a diagram illustrating an example in which a target molecule capturing particle 115 is further bonded to the complex in FIG. 5E. A specific binding substance 116, which recognizes an epitope different from epitopes recognized by the specific binding substance 190 and the specific binding substance 191, is immobilized on a target molecule capturing particle 115.

As described above, by using a target molecule capturing particle, it becomes easy to accommodate, for example, 0 molecules or 1 molecule of the target molecule in each well forming a micro compartment, and to perform digital measurement.

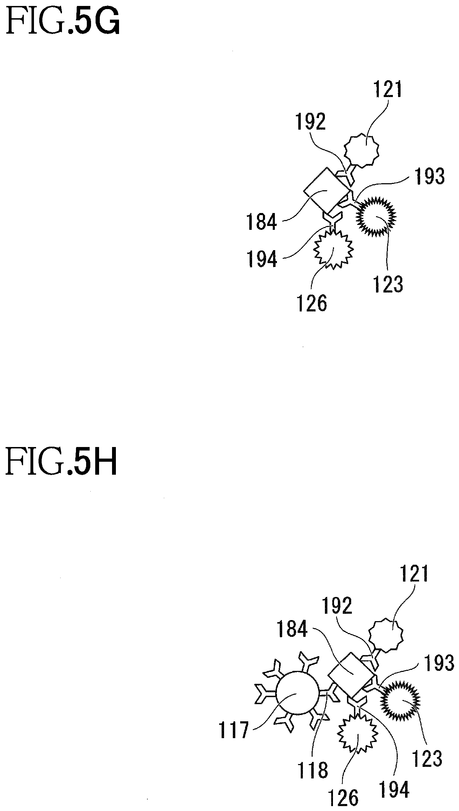

In FIG. 5G, a specific binding substance 192, a specific binding substance 193 and a specific binding substance 194 are bound to a target molecule 184 in order to detect the target molecule 184. The specific binding substance 192 has a label 121. Moreover, the specific binding substance 193 has a label 123. Furthermore, the specific binding substance 194 has a label 126. The specific binding substance 192, the specific binding substance 193 and the specific binding substance 194 each recognize a different epitope. In addition, the label 121, the label 123 and the label 126 can be distinguished from each other.

FIG. 5H is a diagram illustrating an example in which a target molecule capturing particle 117 is also bonded to the complex in FIG. 5G. A specific binding substance 118 which recognizes an epitope different from epitopes recognized by the specific binding substance 192, the specific binding substance 193, and the specific binding substance 194, is immobilized on a target molecule capturing particle 117. As described above, by using a target molecule capturing particle, it becomes easy to accommodate, for example, 0 molecules or 1 molecule of the target molecule in each well forming a micro compartment, and to perform digital measurement.

In this way, in FIGs. 5A, 5C, 5E and 5G (or FIGs. 5B, 5D, 5F and 5H), since combinations of labels bonded to target molecules are different from each other, it is possible to identify the target molecules 100, 180, 182 and 184 mixed and present in the sample and simultaneously detect the target molecules with high accuracy. The number of target molecule identifiable at the same time types depends on the number of combinations of distinguishable label types, and can be further increased.

Note that in the examples of FIGs. 5A to 5H of the method for detecting a target molecule according to the above-described second embodiment, two or more labels are bound to one target molecule; however, uses that take into consideration the case in which one label is bound to one target molecule are also possible.

The method for detecting a target molecule according to the present embodiment provides a method for detecting a target molecule, including: a step of preparing a plurality of types of specific binding substances which are labeled with a plurality of nucleic acid fragments each having a labeling substance that generates fluorescence signals of different fluorescence wavelengths and each recognize a different epitope, and a plurality of types of first target molecules; a step of binding the plurality of types of the specific binding substances to the plurality of types of the first target molecules; a step of respectively detecting the plurality of the specific binding substances; and a step of determining the plurality of types of the first target molecules by a plurality of the fluorescence signals corresponding to the plurality of types of the specific binding substances.

In the method for detecting a target molecule according to this embodiment, a second target molecule having at least one of the plurality of types of the first target molecules is prepared, and the second target molecule may be detected via detection of at least one of the plurality of types of the first target molecules.

The method for detecting a target molecule according to this embodiment can be applied to the analysis of, for example, a protein, an extracellular vesicle (Exosome), an endoplasmic reticulum, a virus, a cell and the like as a target molecule, and it can also be applied to analysis or the like of surface membrane proteins of an endoplasmic reticulum, a virus, cells or the like.

More specifically, the method for detecting a target molecule according to the present embodiment can be applied to the detection of representative biomarkers in liquid biopsies, such as extracellular vesicles (Exosomes), and the like, and the detection of proteins on the membrane surface of exosomes.

Exosomes are biomarkers deeply involved in various situations such as onset, malignization, progression, and metastasis of diseases typified by cancer.

In addition, an exosome is a membrane vesicle having a diameter of about 40 nm to 150 nm and secreted by most cells.

Exosomes are observed in body fluids such as saliva, blood, urine, amniotic fluid, malignant ascites and the like, and are also secreted from cultured cells in the body. In recent years, it has been pointed out that exosomes may play a role in transmitting information to distant cells and tissues. Exosomes contain various proteins, lipids, and nucleic acids, and are thought to cause functional changes and physiological changes by being transported to other cells. As a specific example, it has been suggested that exosomes have roles such as mediating adaptive immune responses against infectious pathogens and tumors, tissue repair, neurotransmission and delivery of pathogenic proteins.

Moreover, in recent studies, it has been known that proteins present on the membrane surface of exosomes vary depending on a person or the type of cancer, and the distribution of these proteins in a biological sample also depends on the type of cancer. As proteins present on the membrane surface of exosomes, for example, it is known that proteins such as CD9, CD63, CD81, and the like, which are biomarkers of exosomes, are present.

Therefore, by investigating the distribution and behavior of proteins such as CD9, CD63, CD81, and the like on the surface of these exosome membranes, there is a possibility that exosomes can be applied to the investigation and examination of the type of cancer, the possibility of cancer development, the degree of cancer progression, and the like.

According to the method for detecting a target molecule according to the present embodiment, by using a labeled specific binding substance (for example, an antibody) that specifically binds to a protein such as CD9, CD63, CD81 or the like appearing on the membrane surface of one exosome, it is possible to detect proteins on the surface of the exosome membrane.

For example, by configuring the label bound to each antibody to emit different fluorescence signals in an antibody that recognizes an epitope of CD9, in an antibody that recognizes an epitope of CD63, and in an antibody that recognizes an epitope of CD81, it is possible to detect the presence of exosomes and to determine the presence or absence and distribution of the presence of CD9, CD63, and CD81 on the exosome membrane surface according to the color of multicolors.

For example, exosomes having one of CD9, CD63, and CD81, and exosomes having two of exosomes CD9, CD63, and CD81, and exosomes having three of CD9, CD63, and CD81 can be analyzed and determined by color difference, and the distribution of each exosome can be observed by color difference.

In other words, with the method for detecting a target molecule according to the present embodiment, it is possible to observe the distribution and behaviors of a protein as a biomarker present on the membrane surface of exosomes in a biological sample, and it is possible to apply this to quick and accurate qualitative analysis and quantitative analysis of each protein.

An example of applying the method of detecting a target molecule according to the present embodiment to the analysis of a protein on the surface of an exosome is described above, the method is not limited to the above example, and it is also possible to apply the method to analysis of a protein present on the membrane surface of viruses, cells, and the like, and to the evaluation and detection of biomarkers indicating the possibility of cancer, and the like.

### [Target Molecule Detection Kit]

One embodiment of the present invention, not covered by the claimed invention, provides a target molecule detection kit that includes a plurality of types of specific binding substances that are specific binding substances for a target molecule, each recognize a different epitope, and are labeled so as to distinguishable from each other.

In the kit according to the present embodiment, the target molecule, specific binding substances and labels are the same as those described above. The specific binding substances may be labeled with different nucleic acid fragments.

### Examples

The present invention will be hereinafter described in further detail based on examples. The present invention is not limited to any of these examples.

### [Experimental Example 1]

### (Preparation of a Target Molecule Capturing Particle (PSA))

Target molecule capturing particles were prepared for capturing a prostate-specific antigen (PSA), which is one kind of a prostate cancer marker, as a target molecule.

First, an anti-PSA monoclonal antibody (type " 1H12", Hytest Ltd.) was biotinylated. For biotinylation, a commercially available kit (trade name "EZ-Link Sulfo-NHS-LC-Biotin", Thermo Fisher Scientific Inc.) was used. More specifically, Sulfo-NHS-LC-Biotin (500 µL of 0.2 mg/mL) was added to an anti-PSA monoclonal antibody diluted with a phosphate buffer solution (PBS) in a 1.5 mL tube, where a molar amount of Sulfo-NHS-LC-Biotin was 50 times that of the anti-PSA monoclonal antibody. Then, the mixture was allowed to stand on ice for 2 hours, and biotinylation of the antibody was performed.

Next, in order to remove unreacted Sulfo-NHS-LC-Biotin and reaction by-products from the mixed solution after the reaction, a gel filtration column (type "Zeba Spin Desalting Column", Thermo Fisher Scientific Inc.) was used, and a biotinylated antibody was purified. After column purification, the amount of protein was measured by BCA (bicinchoninic acid) assay, and the yield was determined.

Then, dispersed streptavidin-labeled beads (type "Magnosphere", JSR Corporation) were mixed with the biotinylated anti-PSA antibody, mixed for 10 minutes at room temperature, and biotin and streptavidin were bound in a 1 × binding buffer. Then, the beads were set in a magnetic stand for 1 minute, the beads were collected and the supernatant was discarded, and the beads were then washed three times with a 1 × binding buffer to obtain target molecule capturing particles.

### [Experimental Example 2]

### (Preparation of Target Molecule Capturing Particles (CEA))

Target molecule capturing particles were prepared for capturing carcinoembryonic antigen (CEA), which is one kind of a digestive system cancer marker, as a target molecule. Target molecule capturing particles were prepared in the same manner as in Experimental Example 1 except that anti-CEA monoclonal antibody (type " 10-1392", Fitzgerald) was used as the antibody.

### [Experimental Example 3]

### (Preparation of Nucleic Acid-labeled Anti-PSA Antibody)

A DNA fragment (DNA fragment 1) having the base sequence indicated in sequence No. 1 and a DNA fragment (DNA fragment 2) having the base sequence indicated in sequence No. 2 were respectively bonded to an anti-PSA monoclonal antibody (type "5A6", Hytest Ltd.) and an anti-PSA monoclonal antibody (type "8A6", Hytest Ltd.) to prepare a nucleic acid-labeled anti-PSA antibody. A commercially available kit (trade name "Protein-Oligo Conjugation Kit", Solulink, Inc.) was used for binding DNA fragments. The nucleic acid-labeled anti-PSA antibody was prepared so that 5 to 20 times as much nucleic acid as that of the antibody (one molecule of antibody) was modified.

### [Experimental Example 4]

### (Preparation of Nucleic Acid-labeled Anti-CEA Antibody)

In the same manner as in Experimental Example 3, a DNA fragment having the base sequence indicated in sequence No. 3 (DNA fragment 3) and a DNA fragment having the base sequence indicated in sequence No. 4 (DNA fragment 4) were respectively bonded to an anti-CEA monoclonal antibody (type "10-1393", Fitzgerald) and anti-CEA monoclonal antibody (type "10-7883", Fitzgerald) to prepare a nucleic acid-labeled anti-CEA antibody.

The nucleic acid-labeled anti-CEA antibody was prepared so that 5 to 20 times as much nucleic acid as that of the antibody (one molecule of antibody) was modified.

### [Experimental Example 5]

### (Preparation of a Sample Containing a Target Molecule)

As a target molecule, PSA (type "PSA calibrator", Sysmex Corporation) and CEA (type "HISCL CEA calibrator", Kaynos) were diluted with 5% bovine serum albumin (BSA) -1 × PBS solution to concentrations of 0, 0.01, 0.05, 0.1, and 0.5 ng/mL, respectively, to prepare a sample including a target molecule.

### [Experimental Example 6]

### (Preparation of a Detection Device)

CYTOP (registered trademark) (manufactured by Asahi Glass Co., Ltd.) was spin-coated on a 0.5 mm thick glass substrate and baked at 180°C for 1 hour. The thickness of the formed CYTOP was 3 µm.

Then, a positive photoresist was coated and a well pattern was formed using a photomask. After that, the CYTOP was dry etched by O2 plasma. Next, in order to remove the photoresist remaining on the surface, the surface was washed and rinsed with acetone and ethanol.

By the above operation, a 100-block well array was formed in a specified area on the substrate. Moreover, each block had 10,000 wells. Therefore, a total of one million wells were formed in the above-mentioned area. In addition, the diameter of a well (micro compartment) formed by CYTOP was 5 µm, and had a volume such that signal detection by an Invader reaction was possible in several minutes.

Then, a glass plate (lid) having a liquid-feeding port and the above-described substrate were adhered using a double-sided tape having a thickness of 100 µm and processed into a channel shape to make a detection device. The glass plate and the substrate were adhered so that the area where the above-described wells were formed was arranged in the flow path.

### [Example 1]

### (PSA Detection 1)

Experiments were performed using PSA as a target molecule. 50 µL of solutions containing various concentrations of PSA were respectively added to a sample tube (non-protein adsorption, Eppendorf). Then, 106 target molecule capturing particles, 50 µL of 10 µg/mL nucleic acid-labeled anti-PSA antibody labeled with a DNA fragment having a base sequence indicated in sequence No. 1, and 50 µL of 10 µg/mL nucleic acid-labeled anti-PSA antibody labeled with a DNA fragment having a base sequence indicated in sequence No. 2 were respectively added to each tube, and the mixture was reacted at room temperature for 1 hour while stirring on a shaker to form a complex.

Next, in order to remove unreacted nucleic acid-labeled anti-PSA antibody, centrifugation, removal of the supernatant, and addition of PBS-T (PBS containing 0.05% Tween) were repeatedly performed three times for washing, and finally the supernatant was removed to obtain a complex.

Then, 20 µL of a solution obtained by mixing the complex and Invader reaction reagent was fed to the liquid-feeding port of the detection device prepared in Experimental Example 6. As a result, a part of the 20 µL of solution was distributed in the wells on the substrate. Invader reaction reagents contained 1 µM allele probe 1 (sequence No. 5), 1 µM allele probe 2 (sequence No. 6), 1 µM Invader oligo 1 (sequence No. 7), 1 µM FRET Cassette (FAM) (manufactured by Hologic, Inc.), 1 µM FRET Cassette (Redmond RED) (manufactured by Hologic, Inc.), 10 mM MOPS (pH 7.5), 6.25 mM MgCl₂, 50 U/µL Cleavase and Tween 20.

The allele probe 1 hybridizes with the DNA fragment of sequence No. 1 bound to the nucleic acid-labeled anti-PSA antibody, and as a result of the Invader reaction, the fluorescent dye FAM emits fluorescence.

In addition, the allele probe 2 hybridizes with the DNA fragment of sequence No. 2 bound to the nucleic acid-labeled anti-PSA antibody, and as a result of the Invader reaction, the fluorescent dye Redmond RED emits fluorescence.

The observation of fluorescence of both FAM and Redmond RED in a well of the detection device indicates that at least one molecule of PSA is contained in the well.

Then, 100 µL of FC-40 (Sigma Corporation) as a fluorine-based oil was delivered as a sealing solution. In doing so, the upper part of each well was covered with the sealing solution. As a result, each well became an independent reaction chamber in which the above-described complex and the Invader reaction reagent were encapsulated. Next, the Invader reaction was performed by heating on a hot plate at 63°C for 15 minutes.

Then, the fluorescence of FAM and Redmond RED in each well was detected using a fluorescence microscope (manufactured by Olympus Corporation). The exposure time was 2000 milliseconds.

As a result, it became clear that PSA diluted to a concentration of 0.01 ng/mL with 5% BSA-1 × PBS solution was detectable. In addition, the heating time required for the Invader reaction in this example was 15 minutes, so it became clear that PSA could be detected rapidly.

### [Comparative Example 1]

Similar experiments were performed using the PSA solution used in Example 1; however, as the nucleic acid-labeled PSA antibody, only 10 µg/mL of the nucleic acid-labeled anti-PSA antibody labeled with the DNA fragment having the base sequence indicated in sequence No. 1 was used.

As a result, it became clear that, due to misrecognition by the anti-PSA antibody, the lower limit of the detectable PSA concentration is a higher value than the lower limit of the PSA concentration that could be detected in Example 1.

### [Example 2]

### (PSA Detection 2)

Experiments were performed using PSA as a target molecule. The same reaction as in Example 1 was performed except that the concentration of PSA in the sample was changed to 0 pg/mL (0 fM), 0.1 pg/mL (3 fM), 1 pg/mL (30 fM), and 10 pg/mL (300 fM), and fluorescence of both FAM and Redmond RED was detected.

As described above, the observation of both FAM and Redmond RED fluorescence in the wells of the detection device indicates that at least one molecule of PSA is contained in the well.

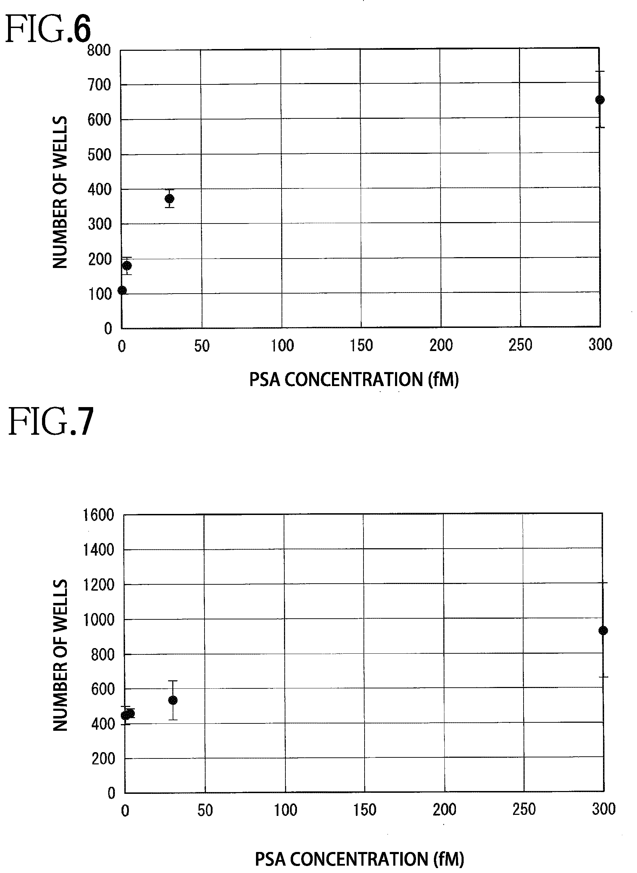

FIG. 6 is a graph illustrating the results of measuring the number of wells in which fluorescence of both FAM and Redmond RED was observed from the reaction of each concentration of PSA sample. As a result, it became clear that PSA solutions at concentrations of 0 pg/mL (0 fM) and 0.1 pg/mL (3 fM) can be clearly distinguished.

Moreover, in this example, the heating time required for the Invader reaction was 15 minutes, so it became clear that PSA can be detected rapidly.

### [Comparative Example 2]

For the wells of the device in which PSA was detected in Example 2, only the number of wells in which fluorescence of FAM was observed was measured. As a result, it is possible to obtain a similar result as when PSA is detected using only one type of anti-PSA antibody.

FIG. 7 is a graph illustrating the results of measuring the number of wells in which fluorescence of FAM was observed from the reaction of each concentration of PSA sample. As a result, it was difficult to clearly distinguish PSA solutions at concentrations of 0 pg/mL (0 fM), 0.1 pg/mL (3 fM) and 1 pg/mL (30 fM).

This result indicates that, due to misrecognition by anti-PSA antibody, the lower limit of detectable PSA concentration is a much higher value than the lower limit of PSA concentration detectable in Example 2. In other words, by binding a plurality of types of specific binding substances to a target molecule and detecting two or more types of specific binding substances, even in the case where a non-specific binding substance causes misrecognition of a target molecule, the accuracy of detection of a target molecule can be markedly improved. Moreover, according to this example, PSA can be quickly detected compared with a conventional method. It is thought that this is because the nucleic acid-labeled anti-PSA antibody was prepared so that a plurality of nucleic acids serving as the reaction origin of the fluorescence signal were modified with respect to the antibody (one molecule of the antibody). Furthermore, it is considered that by providing a configuration such that target molecules were individually captured in each well of the device, even when a large number of labeled nucleic acids were present, the labeled nucleic acids did not interfere with each other, and detection of the target molecule was possible.

### [Example 3]

### (PSA and CEA Detection)

Experiments were conducted using a mixture of PSA and CEA as the target molecule. 50 µL of a solution in which 0.01 ng/mL PSA and 0.01 ng/mL CEA were mixed at a volume ratio of 1 : 2 was added to a sample tube (non-protein adsorption, Eppendorf). Then, 106 target molecule capturing particles, 50 µL of 10 µg/mL nucleic acid-labeled anti-PSA antibody labeled with a DNA fragment having the base sequence indicated in sequence No. 1, 50 µL of of 10 µg/mL nucleic acid-labeled anti PSA antibody labeled with a DNA fragment having the base sequence indicated in sequence No. 2, 50 µL of of 10 µg/mL nucleic acid-labeled anti PSA antibody labeled with a DNA fragment having the base sequence indicated in sequence No. 3, and 50 µL of of 10 µg/mL nucleic acid-labeled anti PSA antibody labeled with a DNA fragment having the base sequence indicated in sequence No. 4 were added to the tube, and the mixture was reacted at room temperature for 1 hour while stirring on a shaker to form a complex.

The nucleic acid-labeled anti-PSA antibody was prepared so that 5 to 20 times as much nucleic acid as that of the antibody (one molecule of antibody) was modified.

In addition, the nucleic acid-labeled anti-CEA antibody was prepared so that 5 to 20 times as much nucleic acid as that of the antibody (one molecule of antibody) was modified.

Then, 20 µL of a solution obtained by mixing the complex and Invader reaction reagent was fed to the liquid-feeding port of the detection device prepared in Experimental Example 6. Invader reaction reagents contained 1 µM allele probe 1 (sequence No. 5), 1 µM allele probe 2 (sequence No. 6), 1 µM allele probe 3 (sequence No. 8), 1 µM allele probe 4 (sequence No. 9), 1 µM Invader oligo 2 (sequence No. 10), 1 µM FRET Cassette (FAM) (manufactured by Hologic, Inc.), 1 µM FRET Cassette (Redmond RED) (manufactured by Hologic, Inc.), 1 µM FRET Cassette (TAMRA) (manufactured by Hologic, Inc.), 10 mM MOPS (pH 7.5), 6.25 mM MgCl₂, 50 U/µL Cleavase and Tween 20.

The allele probe 1 hybridizes with the DNA fragment of sequence No. 1 bound to the nucleic acid-labeled anti-PSA antibody, and as a result of the Invader reaction, the fluorescent dye FAM emits fluorescence.

In addition, the allele probe 2 hybridizes with the DNA fragment of sequence No. 2 bound to the nucleic acid-labeled anti-PSA antibody, and as a result of the Invader reaction, the fluorescent dye Redmond RED emits fluorescence.

Moreover, the allele probe 3 hybridizes with the DNA fragment of sequence No. 3 bound to the nucleic acid-labeled anti-CEA antibody, and as a result of the Invader reaction, the fluorescent dye FAM emits fluorescence.

Furthermore, the allele probe 4 hybridizes with the DNA fragment of sequence No. 4 bound to the nucleic acid-labeled anti-CEA antibody, and as a result of the Invader reaction, the fluorescent dye TAMRA emits fluorescence.

The observation of fluorescence of both FAM and Redmond RED in a well of the detection device indicates that at least one molecule of PSA is contained in the well. In addition, the observation of fluorescence of both FAM and TAMRA in a well of the detection device indicates that at least one molecule of CEA is contained in the well.

Then, 100 µL of FC-40 (Sigma Corporation) as a fluorine-based oil was delivered as a sealing solution. Next, the Invader reaction was performed by heating on a hot plate at 63°C for 15 minutes.

Then, the fluorescence of FAM, Redmond RED and TAMRA in each well was detected using a fluorescence microscope (Olympus Corporation). The exposure time was 2000 milliseconds.

As a result, the concentration ratio of PSA and CEA calculated from the number of chambers in which fluorescence of both FAM and Redmond RED was observed and the number of chambers in which fluorescence of both FAM and TAMRA was observed was calculated as 1 : 2. In addition, the heating time required for the Invader reaction in this example was 15 minutes, and it became clear that PSA and CEA can be detected quickly.

### [Example 4]

### <Reagent Preparation>

### (Exosome-containing sample)

A culture supernatant that contains an exosome, prepared by culturing colorectal cancer cell line HCT 116 for 3 days in a medium in which McCoy's 5A Medium (Lonza) : Exo-FBS (SBI) : penicillin streptomycin solution (Wako Pure Chemical Industries, Ltd.) are mixed at 90 : 10 : 1, was used. The culture supernatant was adjusted to 300 × g, and cells were removed after 10 minutes. Moreover, the culture supernatant was adjusted to 2000 × g, and debris was removed after 10 minutes. Furthermore, the culture supernatant was adjusted to 10000 × g, and even finer debris was removed after 30 minutes. Finally, the culture supernatant was filtered through a 0.22 µm filter unit (Millipore Corporation).

Human serum (human serum AB, produced by Kohjin Bio Co., Ltd.) was used for serum that includes exosome.

### (Preparation of Antibody-Immobilized Magnetic Beads)

In order to immobilize the antibody on magnetic beads, an anti-exosome antibody (type "CD9", Cosmo Bio Co., Ltd.) was added to a carboxyl group-modified magnetic bead (Magnosphere, LC 300, JSR Corporation) solution and mixed to 100 µL, the mixture was reacted with a rotator for 30 minutes, then a condensing agent EDC was further added, and the reaction was carried out for 3 hours to immobilize the antibody on the carboxyl magnetic beads.

In order to remove unreacted antibody and reagent after the reaction, antibody-immobilized carboxyl magnetic beads were magnetically captured using a magnetic stand and repeatedly washed three times with PBS-T (PBS containing 0.1% Tween 20) to prepare antibody-immobilized carboxylic magnetic beads (CD9 beads).

### (Antibody Modifying Nucleic Acid and Detecting Nucleic Acid)

As nucleic acids for antibody modification and a nucleic acid detection reaction, the following nucleic acids were used.
- DNA fragment 5: has the base sequence indicated in sequence No. 11.
- DNA fragment 6: has the base sequence indicated in sequence No. 12.
- Allele probe 3: has the base sequence indicated in sequence No. 8.
- Allele probe 5: has the base sequence indicated in sequence No. 13.
- Invader Oligo 2: has the base sequence indicated in sequence No. 10.

### (Preparation of Nucleic Acid-modified Antibodies)

In the same manner as in Experimental Example 3, DNA fragments 5 and 6 were bonded to two types of anti-exosome antibodies (type "CD 63", type "CD 81", Cosmo Bio Co., Ltd.), and nucleic acid modified antibodies (DNA fragment 5 - CD 63, DNA Fragment 6 - CD 81) were prepared.

The nucleic acid-modified antibodies were prepared so that each of the DNA fragment 5-CD 63 and the DNA fragment 6-CD 81 was modified to 5 to 20 times the amount of nucleic acid with respect to the antibody (one molecule of the antibody).

### (Preparation of Nucleic Acid Detection Reagent)

In order to detect the nucleic acid-modified antibodies by a digital Invader reaction, an Invader reaction reagent was prepared as a nucleic acid detection reagent. The reagent composition of the Invader reaction reagent in this example is as follows.

The Invader reaction reagents in Example 4 contained 0.1 µM allele probe 3 (sequence No. 8), 0.1 µM allele probe 5 (sequence No. 13), 1 µM Invader Oligo 2 (sequence No. 10), 2 µM FRET Cassette (FAM) (produced by Hologic, Inc.), 2 µM FRET Cassette (Redmond RED) (produced by Hologic, Inc.), 10 mM MOPS (pH 7.9), 10 mM MgCl₂, and 1000 U/ µL Cleavase.

Note that the concentration of each component in these Invader reaction reagents is the final concentration in the Invader reaction reagent according to Example 4.

### <Reaction of Antigen, Antibody-immobilized Beads and Nucleic Acid-modified Antibodies>

In a sample tube, 106 antibody-immobilized carboxylic magnetic beads (CD9 beads), culture supernatants or serum each adjusted so as to become 0 or 50%, and two types of nucleic acid-modified antibodies (DNA Fragment 5 - CD 63, DNA fragment 6 - CD 81) adjusted to 0.1 ng/mL were mixed so that the total amount was 100 µL, and the mixture was reacted at room temperature for 3 hours on a rotator to form a complex.

After the reaction, the magnetic beads obtained were magnetically collected using a magnetic stand, removal of the supernatant and addition of 0.1% Tween-containing PBS (PBS-T) were performed five times for washing, and finally the supernatant was removed to obtain a complex.

The obtained complex was then washed and diluted with PBS to prepare a reaction mixture 1.

### <Assembling the Flow Cell>

The following three members were assembled so that the height of the flow path was 100 µm and the width was 6 to 7 mm.
- COP chamber (ϕ5 µm micro pores formed at depth of 3.5 µm, manufactured by STRATEC)
   • Double-sided tape (film thickness 100 µm, Hoyo Co., Ltd.)
   • Channel strip for feeding COP (carbon black coloring, with liquid-feed and liquid-discharge ports, Arakawa Jushi Inc.)

### <Feeding Reaction Mixed Solution>

20 µL of a solution obtained by mixing the complex and Invader reaction reagent was fed to the flow cell. Then, 100 µL of FC-40 (Sigma Corporation) was supplied as a sealing solution, and each chamber was sealed.

### <Nucleic Acid Detection Reaction>

The above-described flow cell was set on a hot plate and caused to react at 66°C for 15 minutes.

### <Chamber Observation>

After cooling the flow cell, fluorescence of FAM and Redmond RED in each well was detected with a fluorescence microscope BZ-710 (KEYENCE Corporation). The exposure time for FAM was set to 1200 msec, and for Redmond RED was set to 1000 msec.

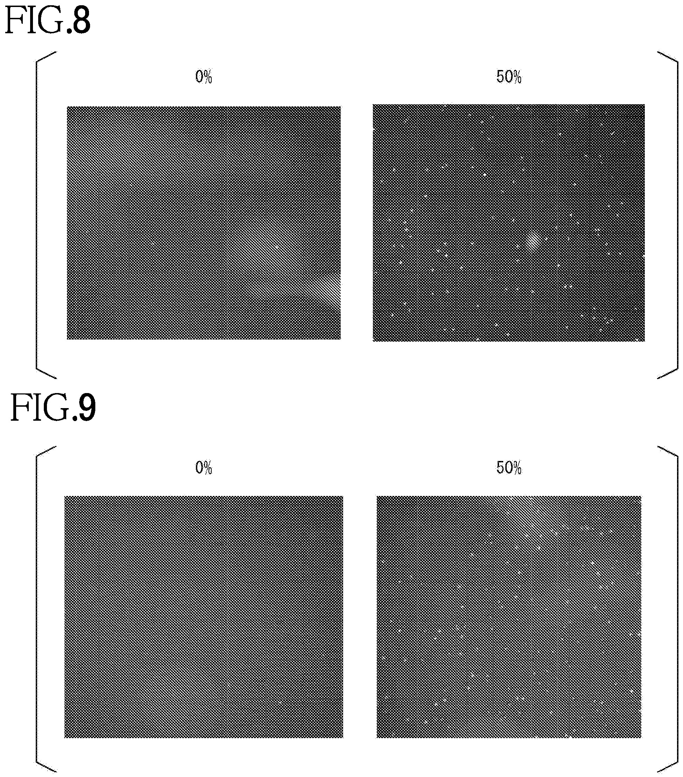

The reaction in the sample solution containing 50% serum was observed with a fluorescence microscope, and the results of superimposing the respective fluorescence images are illustrated on the right side of FIG. 9. Note that the left side of FIG. 9 is an image in the case where serum is not added (serum 0%).

As a result, it was confirmed that the number of fluorescent chambers varied depending on the content ratio of culture supernatant and serum in the sample.

From these results, it was indicated that the exosome membrane surface protein was recognized by the digital Invader method using the exosome antibody, and the exosomes contained in the culture supernatant and the serum could be detected.

Moreover, in the reaction using each sample-containing solution, the number of chambers filled with magnetic beads in one block, the number of chambers in which both magnetic beads and fluorescence signals were detected, and the number of chambers in which only fluorescence signals were detected were counted are illustrated in Table 1.

**[Table 1]**

| Exosome Sample Content (%) | Wells with Beads | Wells in which Beads Entered and Fluorescence was Observed | Wells in which Fluorescence was Observed without Beads Entering |
|---|---|---|---|
| 0% | 107 | 5 | 0 |
| Culture supernatant 50% | 382 | 205 | 1 |
| Serum 50% | 427 | 233 | 2 |

As illustrated in Table 1, for the case of the culture supernatant and serum, most of the fluorescence signals detected overlapped with the beads, indicating that exosomes were detected on magnetic beads.

Note that the allele probe 3 hybridizes with the DNA fragment 5 of sequence No. 11 bound to CD 63, and as a result of the Invader reaction, the fluorescent dye FAM emits fluorescence.

For example, on the right side of FIG. 8 and the right side of FIG. 9, FAM emitted green fluorescence.

Moreover, it is considered that the FAM fluorescence was observed because CD63, which is a protein present on the exosome membrane surface, was detected.

In addition, the allele probe 5 hybridizes with the DNA fragment 6 of sequence No. 12 bound to CD 81, and as a result of the Invader reaction, Redmond RED, which is a fluorescent dye, emits fluorescence.

For example, on the right side of Fig. 8 and the right side of Fig. 9, Redmond RED emitted red fluorescence.

Moreover, it is considered that fluorescence of Redmond RED was observed because CD81, which is a protein present on the exosome membrane surface, was detected.

Furthermore, on the right side of FIG. 8 and the right side of FIG. 9, yellow fluorescence, which is considered as a fluorescence signal when fluorescence of both green fluorescence derived from FAM and red fluorescence derived from Redmond RED are obtained, was observed.

In addition, it is considered that the observance of fluorescence (yellow) derived from both FAM and Redmond RED means that of the exosomes it was possible to detect exosomes in which the two proteins CD63, CD81 are present on the exosome membrane surface.

Furthermore, from the results of the fluorescence image on the right side of FIG. 8 and the fluorescence image on the right side of FIG. 9, it is considered that from the green, red and yellow fluorescence signals, it was possible to confirm the presence of exosomes having CD63 on the surface of the membrane, exosomes having CD81 on the surface of the membrane, and exosomes having both CD63 and CD81 on the membrane surface.

In other words, it was confirmed that exosomes as the second target molecule can be detected through the detection of CD63 and CD81 as a plurality of types of first target molecules.

Moreover, according to this example, it is considered that from the detection of a plurality of fluorescence signals such as green, red and yellow, the distribution of CD 63 and CD81 present in exosomes in a biological sample could be determined.

Furthermore, according to the present embodiment, it is considered that by detecting the distribution of a plurality of fluorescence signals such as green, red, and yellow, the distribution of exosomes having CD63 on the membrane surface, exosomes having CD81 on the membrane surface, and exosomes having both CD63 and CD81 on the membrane surface could be observed in the biological sample.

Since a plurality of proteins on the surface of the exosome membrane surface were recognized and detected by the digital Invader reaction with the nucleic acid-modified antibody, it is indicated that, even in the case of having many reaction initiating substances (case such as in this example where an antibody is modified by many nucleic acid fragments, or the case in which a lot of substances serving as the base points of the fluorescence signals are bound), quantitative detection of a plurality of items can be performed in a short time by digital measurement.

This is because a large number of fluorescently labeled nucleic acid fragments are bound to an antibody that recognizes protein.

In addition, in this example, since the size of a well was configured so that one or less exosome (second target molecule having a protein as the first target molecule) was introduced into one well, it is considered that proteins possessed by exosomes could be detected without interference or the like of fluorescence derived from surplus antibodies even when many antibodies having fluorescently labeled nucleic acid fragments are present in the flow cell.

On the other hand, in the conventional digital PCR that amplifies a target nucleic acid, the reaction time required for detection is large, so detection in a short time is difficult.

Moreover, in conventional flow cytometry in which an antibody is fluorescently labeled, since exosomes and proteins that will be a target are small, they need to be separated using fluorescent labeled beads or the like for detection, and highly sensitive detection is difficult.

Furthermore, in conventional immuno-PCR using a nucleic acid-modified antibody, the amount of labeled nucleic acid for detection needs to be controlled, and quantitative detection is difficult.

As described above, according to the conventional method, detecting a target molecule quickly and accurately is considered to be difficult.

According to the method for detecting a target molecule according to the present embodiment, it is indicated that even in the case where a biological sample flows to one flow cell, quantitative detection of target molecules of a plurality of items is possible in a short time by multicolor detection that simultaneously detects a plurality of fluorescence signals.

In addition, according to the method for detecting a target molecule according to this embodiment, it is possible to detect proteins and the like present on the surface of a biological sample such as exosomes or the like, and by multicolor detection, it is also considered to be possible to apply this method to qualitative analysis such as observing the distribution of these proteins and the like, investigating the behavior and distribution of proteins and the like that are biomarkers related to cancer, and the like.

### Industrial Applicability

According to the present invention, even in the case where a non-specific binding substance causes misrecognition of a target molecule, it is possible to provide a technique capable of accurately detecting a target molecule.

### [Reference Signs List]

100, 180, 182, 184...Target molecule
110, 112, 113, 115, 117...Target molecule capturing particle
111, 114, 116, 118, 120, 122, 124, 125, 190, 191, 192, 193, 194 ...Specific binding substance
121, 123, 126... Label (labeling molecule)
130, 150, 160, 170...Complex
140...Molecule that is not a target molecule
200...Detection device
210...Substrate
220...Flow channel
230...Lid
240...Well
250...Signal

## Claims

1. A method for detecting a target molecule, comprising:
a step of preparing a plurality of types of specific binding substances which are labeled with a plurality of nucleic acid fragments each having a labeling substance that generates a fluorescence signal of a different fluorescence wavelength, and which each recognize a different epitope, and preparing a first target molecule;
a step of binding the plurality of types of specific binding substances to the first target molecule;
a step of respectively detecting the plurality of types of specific binding substances; and
a step of determining the first target molecule by a plurality of the fluorescence signals corresponding to the plurality of types of specific binding substances;
wherein a second target molecule having at least one of the first target molecule is prepared; and
the second target molecule is detected via detection of at least one of the first target molecule;
wherein the plurality of types of specific binding substances is detected by using Invader method

2. The detection method according to claim 1, wherein that a plurality of types of the first target molecule are detected.

3. The detection method according to claims 1 or 2, wherein determining that the first target molecule is present if two or more types of the plurality of types of specific binding substances are detected.

4. The detection method according to any one of claims 1 to 3, wherein several to several tens of the plurality of nucleic acid fragments are bound to one molecule of the specific binding substance.

5. The detection method according to any one of claims 1 to 4, wherein the specific binding substance is an antibody.

6. The detection method according to claim 1, wherein the second target molecule is an exosome.

7. The detection method according to claim 6, wherein at least one of the first target molecule is present on a membrane surface of the exosome.

8. The detection method according to claims 6 or 7, wherein each of the first target molecule is a disease marker protein.

9. The detection method according to claim 1, further comprising
a step of
preparing a detection device including a substrate having a plurality of wells, and
introducing the second target molecule into a respective well of the plurality of wells after the step of binding the plurality of types of specific binding substances to the first target molecule.

10. The detection method according to claim 9, wherein the volume of the well is 100 picoliters or less.

11. The detection method according to any one of claims 1 to 10, wherein detection of the target molecule is performed in units of one molecule.

12. The detection method according to any one of claims 1 to 11, wherein the step of respectively detecting the plurality of types of specific binding substances includes a signal amplification reaction.

## Patentansprüche

1. Verfahren zum Nachweis eines Zielmoleküls, umfassend:
einen Schritt der Herstellung einer Vielzahl von Typen spezifischer Bindungssubstanzen, die mit einer Vielzahl von Nukleinsäurefragmenten markiert sind, die jeweils eine Markierungssubstanz aufweisen, die ein Fluoreszenzsignal mit einer unterschiedlichen Fluoreszenzwellenlänge erzeugt, und die jeweils ein unterschiedliches Epitop erkennen, und die Herstellung eines ersten Zielmoleküls;
einen Schritt des Bindens der Vielzahl von Typen spezifischer Bindungssubstanzen an das erste Zielmolekül;
einen Schritt des jeweiligen Nachweises der Vielzahl von Typen spezifischer Bindungssubstanzen; und
einen Schritt des Bestimmens des ersten Zielmoleküls durch eine Vielzahl der Fluoreszenzsignale, die der Vielzahl von Typen spezifischer Bindungssubstanzen entsprechen;
wobei ein zweites Zielmolekül, das mindestens eines der ersten Zielmoleküle aufweist, hergestellt wird; und
das zweite Zielmolekül über den Nachweis von mindestens einem der ersten Zielmoleküle nachgewiesen wird;
wobei die Vielzahl von Typen spezifischer Bindungssubstanzen unter Verwendung der Invader-Methode nachgewiesen wird.

2. Nachweisverfahren nach Anspruch 1, wobei eine Vielzahl von Typen des ersten Zielmoleküls nachgewiesen wird.

3. Nachweisverfahren nach Anspruch 1 oder 2, wobei bestimmt wird, dass das erste Zielmolekül vorhanden ist, wenn zwei oder mehr Typen der Vielzahl von Typen spezifischer Bindungssubstanzen nachgewiesen werden.

4. Nachweisverfahren nach einem der Ansprüche 1 bis 3, wobei mehrere bis mehrere Dutzend der Vielzahl von Nukleinsäurefragmenten an ein Molekül der spezifischen Bindungssubstanz gebunden werden.

5. Nachweisverfahren nach einem der Ansprüche 1 bis 4, wobei die spezifische Bindungssubstanz ein Antikörper ist.

6. Nachweisverfahren nach Anspruch 1, wobei das zweite Zielmolekül ein Exosom ist.

7. Nachweisverfahren nach Anspruch 6, wobei mindestens eines der ersten Zielmoleküle auf einer Membranoberfläche des Exosoms vorliegt.

8. Nachweisverfahren nach Anspruch 6 oder 7, wobei jedes der ersten Zielmoleküle ein Erkrankungsmarkerprotein ist.

9. Nachweisverfahren nach Anspruch 1, welches ferner einen Schritt
des Herstellens einer Nachweisvorrichtung, die ein Substrat mit einer Vielzahl von Vertiefungen enthält, und
des Einbringens des zweiten Zielmoleküls in eine entsprechende Vertiefung der Vielzahl von Vertiefungen nach dem Schritt des Bindens der Vielzahl von Typen spezifischer Bindungssubstanzen an die ersten Zielmoleküle umfasst.

10. Nachweisverfahren nach Anspruch 9, wobei das Volumen der Vertiefung 100 Picoliter oder weniger beträgt.

11. Nachweisverfahren nach einem der Ansprüche 1 bis 10, wobei der Nachweis des Zielmoleküls in Einheiten von einem Molekül durchgeführt wird.

12. Nachweisverfahren nach einem der Ansprüche 1 bis 11, wobei der Schritt des jeweiligen Nachweises der Vielzahl von Typen spezifischer Bindungssubstanzen eine Signalverstärkungsreaktion umfasst.

## Revendications

1. Procédé de détection d'une molécule cible, comprenant :
une étape de préparation d'une pluralité de types de substances de liaison spécifiques qui sont marquées avec une pluralité de fragments d'acide nucléique ayant chacun une substance de marquage qui génère un signal de fluorescence d'une longueur d'onde de fluorescence différente, et qui reconnaissent chacun un épitope différent, et de préparation d'une première molécule cible ;
une étape de liaison de la pluralité de types de substances de liaison spécifiques à la première molécule cible ;
une étape de détection respective de la pluralité de types de substances de liaison spécifiques ; et
une étape de détermination de la première molécule cible par une pluralité de signaux de fluorescence correspondant à la pluralité de types de substances de liaison spécifiques ;
dans lequel une seconde molécule cible ayant au moins une parmi la première molécule cible est préparée ; et
la seconde molécule cible est détectée par l'intermédiaire d'une détection d'au moins une parmi la première molécule cible ;
dans lequel la pluralité de types de substances de liaison spécifique est détectée en utilisant un procédé Invader.

2. Procédé de détection selon la revendication 1, dans lequel une pluralité de types de la première molécule sont détectés.

3. Procédé de détection selon les revendications 1 ou 2, comprenant la détermination de la présence de la première molécule cible si deux types ou plus de la pluralité de types de substances de liaison spécifiques sont détectés.

4. Procédé de détection selon l'une quelconque des revendications 1 à 3, dans lequel plusieurs à plusieurs dizaines de la pluralité de fragments d'acide nucléique sont liés à une molécule de la substance de liaison spécifique.

5. Procédé de détection selon l'une quelconque des revendications 1 à 4, dans lequel la substance de liaison spécifique est un anticorps.

6. Procédé de détection selon la revendication 1, dans lequel la seconde molécule cible est un exosome.

7. Procédé de détection selon la revendication 6, dans lequel au moins une parmi la première molécule cible est présente sur une surface de membrane de l'exosome.

8. Procédé de détection selon les revendications 6 ou 7, dans lequel chacune de la première molécule cible est une protéine marqueur de maladie.

9. Procédé de détection selon la revendication 1, comprenant en outre une étape de préparation d'un dispositif de détection incluant un substrat ayant une pluralité de puits, et
introduction de la seconde molécule cible dans un puits respectif de la pluralité de puits après l'étape de liaison de la pluralité de types de substances de liaison spécifiques à la première molécule cible.

10. Procédé de détection selon la revendication 9, dans lequel le volume du puits est de 100 picolitres ou moins.

11. Procédé de détection selon l'une quelconque des revendications 1 à 10, dans lequel la détection de la molécule cible est réalisée en unités d'une molécule.

12. Procédé de détection selon l'une quelconque des revendications 1 à 11, dans lequel l'étape de détection respective de la pluralité de types de substances de liaison spécifique inclut une réaction d'amplification du signal.


### SEQUENCE LISTING

<110> TOPPAN PRINTING CO., LTD.
<120> METHOD OF DETECTING TARGET MOLECULE
<130> PC-23705
<150> JP2016-100231
   <151> 2016-05-19
<150> JP2016-241023
   <151> 2016-12-13
<160> 10
<170> PatentIn version 3.5
<210> 1
   <211> 56
   <212> DNA
   <213> Artificial Sequence
<220>
   <223> label oligo
<400> 1
   ccgaagggca tgagctgcgt gatgagctgc acggtggagg tgaggcagat gcccag 56
<210> 2
   <211> 56
   <212> DNA
   <213> Artificial Sequence
<220>
   <223> label oligo
<400> 2
   ccgaagggca tgagctgcat gatgagctgc acggtggagg tgaggcagat gcccag 56
<210> 3
   <211> 129
   <212> DNA
   <213> Artificial Sequence
<220>
   <223> label oligo
<400> 3
   tggaggtaaa tattttttaa caatttgtca ctgttcctcc ttttgttttc ctttctgtga 60
   gcaatttcac ccaaattgga accatgctgt atacagtttg gtagctggct ttttatgtct 120
   taccattat 129
<210> 4
   <211> 129
   <212> DNA
   <213> Artificial Sequence
<220>
   <223> label oligo
<400> 4
   tggaggtaaa tattttttaa caatttgtca ctgttcctcc ttttgttttc ctttctgtga 60
   gcaatgtcac ccaaattgga accatgctgt atacagtttg gtagctggct ttttatgtct 120
   taccattat 129
<210> 5
   <211> 24
   <212> DNA
   <213> Artificial Sequence
<220>
   <223> allele probe 1
<400> 5
   cgcgccgagg cgcagctcat gccc 24
<210> 6
   <211> 26
   <212> DNA
   <213> Artificial Sequence
<220>
   <223> allele probe 2
<400> 6
   acggacgcgg agtgcagctc atgccc 26
<210> 7
   <211> 19
   <212> DNA
   <213> Artificial Sequence
<220>
   <223> invader oligo 1
<400> 7
   ccaccgtgca rctcatcaa 19
<210> 8
   <211> 28
   <212> DNA
   <213> Artificial Sequence
<220>
   <223> allele probe 3
<400> 8
   cgcgccgagg aattgctcac agaaagga 28
<210> 9
   <211> 32
   <212> DNA
   <213> Artificial Sequence
<220>
   <223> allele probe 4
<400> 9
   atgacgtggc agaccattgc tcacagaaag ga 32
<210> 10
   <211> 22
   <212> DNA
   <213> Artificial Sequence
<220>
   <223> invader oligo 2
<400> 10
   gcatggttcc aatttgggtg at 22
<210> 11
   <211> 75
   <212> DNA
   <213> Artificial Sequence
<220>
   <223> label oligo
<400> 11
   tttgtcactg ttcctccttt tgttttcctt tctgtgagca atttcaccca aattggaacc 60
   atgctgtata cagtt 75
<210> 12
   <211> 75
   <212> DNA
   <213> Artificial Sequence
<220>
   <223> label oligo
<400> 12
   tttgtcactg ttcctccttt tgttttcctt tctgtgagca atctcaccca aattggaacc 60
   atgctgtata cagtt 75
<210> 13
   <211> 30
   <212> DNA
   <213> Artificial Sequence
<220>
   <223> allele probe 5
<400> 13
   acggacgcgg aggattgctc acagaaagga 30